# EUROPEAN PATENT SPECIFICATION

(11) **EP 2 435 054 B1**
(45) Date of publication and mention of the grant of the patent: **08.05.2013**
(21) Application number: 10726331.1
(22) Date of filing: 28.05.2010
(51) Int. Cl.: A61K 33/44, B01J 20/20, B01J 20/28

(54) **ADSORPTION TEST METHOD OF SPHERICAL CARBON ADSORBENT**
ADSORPTIONSTESTVERFAHREN EINES KUGELFÖRMIGEN KOHLENSTOFFADSORBENS
PROCÉDÉ DE TEST D'ADSORPTION D'UN ADSORBANT CARBONÉ SPHÉRIQUE

(30) Priority: 29.05.2009 US 182231 P
(43) Date of publication of application: 04.04.2012
(73) Proprietor: Mitsubishi Tanabe Pharma Corporation, Osaka 541-8505 (JP)
(72) Inventor: YOSHINARI, Tetsuro, Osaka-shi Osaka 541-8505 (JP); YAMADA, Hiroyuki, Osaka-shi Osaka 541-8505 (JP)
(74) Representative: TER MEER - STEINMEISTER & PARTNER GbR
(86) International application number: PCT/JP2010/059485
(87) International publication number: WO 2010/137741

(56) References cited:
- EP-A1- 1 440 692
- GB-A- 2 025 385
- JP-A- 2006 131 461
- MALIK ET AL: "Structured carbon haemoadsorbents for the removal of middle molecular weight toxins" CARBON, ELSEVIER, OXFORD, GB LNKD- DOI:10.1016/J.CARBON.2005.04.038, vol. 43, no. 11, 1 September 2005 (2005-09-01), pages 2317-2329, XP005000940 ISSN: 0008-6223
- RAYMOND VANHOLDER ET AL: "Review on uremic toxins: Classification, concentration, and interindividual variability" KIDNEY INTERNATIONAL, NATURE PUBLISHING GROUP, LONDON, GB LNKD- DOI:10.1046/J.1523-1755.2003.00924.X, vol. 63, no. 5, 1 May 2003 (2003-05-01), pages 1934-1943, XP009088717 ISSN: 0085-2538

## Description

### TECHNICAL FIELD

The present invention relates to a novel method of testing adsorption of spherical carbon adsorbent.

### BACKGROUND ART

Kremezin (registered trade mark) is a spherical microgranular oral adsorbent comprised of highly pure porous carbon developed by Kureha Chemical Industry Co., Ltd. for the purpose of improving uremia in chronic renal failure and delaying introduction of dialysis, namely, a spherical carbon adsorbent for oral administration.

Conventionally, medicinal carbon has been used, utilizing its adsorbability, for adsorbing gas generated by hyperacidity (hyperchylia and the like) or fermentation in the gastrointestinal tract, and adsorption of toxicant. In addition, attempts have been made to remove uremic toxins in chronic renal failure by adsorption of such toxic substances in the gastrointestinal tract by medicinal carbon. However, a clear effect was not obtained, and a problem has occurred since it also adsorbs enzymes, vitamins, mineral substances and the like.

Therefore, Kureha Chemical Industry Co., Ltd. started to develop an oral adsorbent from 1975, which is suitable for a pharmaceutical use, particularly, removal of uremic toxins in chronic renal failure. They have conducted studies of starting materials and production methods in an attempt to 1) reduce conventional problems of difficulty in taking and constipation action of a charcoal powder, 2) enhance adsorption of ionic organic compounds considered to be biological toxic substances, which resist adsorption by a charcoal powder, and 3) reduce adsorption of digestive enzyme and the like easily adsorbed by a charcoal powder, and completed Kremezin.

Therefore, Kremezin is produced under strict control of starting materials and production methods, and is a physical pharmaceutical product having a physical structure considered to significantly influence adsorption of a toxic substance, and efficacy thereof. At present, however, adsorption tests admitted for a substance considered to directly influence the efficacy of a bulk pharmaceutical standard of Kremezin are only DL-β-aminoisobutyric acid adsorption test and α-amylase adsorption test. Thus, it is difficult to say that there is an analysis method capable of sufficiently control the product property, particularly adsorbability, of Kremezin itself.

In February 2004, Kyucal (registered trade mark) and Merckmezin (registered trade mark) were approved as generic drugs of Kremezin. Both of these generic drugs are considered to show equivalent adsorption behavioral properties in the adsorption tests (ionic organic compound (DL-β-aminoisobutyric acid) and digestive enzyme (α-amylase) in vitro) defined to be the bulk pharmaceutical and/or preparation standard of the above-mentioned Kremezin. However, a recent report has documented that the physical properties of Kremezin and the generic drugs may not necessarily be equivalent (non-patent document 1), and it has also been reported that the both may not be equivalent in efficacy (non-patent document 2, non-patent document 3) and the like. Therefore, an analysis method relating to the adsorbability of a spherical carbon adsorbent, which accurately evaluates the generic drugs of Kremezin as equivalents, does not necessarily exist.

### [PRIOR ART REFERENCES]

### [NON-PATENT DOCUMENT]

non-patent document 1: Pharmaceutical Regulatory Science, Vol. 36, No. 11, p. 497-504 (2005).
non-patent document 2: Pharmaceutical Regulatory Science, Vol. 37, No. 6, p. 373-380 (2006).
non-patent document 3: Japanese Journal of Nephrology, Vol. 51, No. 1, p. 51-55 (2009).
JP 2006 131461 A discloses the preparation of orally administrable carbon particles for adsorption of uremic toxins; the adsorption behaviour is tested with test liquids containing indole. GB 2 025 385 A discloses the preparation of activated carbon particles as adsorbent in hemoperfusion; the adsorption behaviour is tested with test liquids containing creatinine as a small uremic toxin and Vitamin B12 as a representative of a *"middle molecule"* uremic toxin. EP 1 440 692 A1 discloses the preparation of activated carbon particles as adsorbent in hemoperfusion; the adsorption behaviour is tested with test liquids containing putrescine. MALIK et al., CARBON, vol. 43, n° 11, 1 September 2005, p. 2317 - 2329 discloses the preparation of porous activated carbon particles for use as haemoadsorbent; the adsorption behaviour is tested with test liquids containing Interleukin-1ß as uremic toxin.

### DISCLOSURE OF THE INVENTION

### PROBLEMS TO BE SOLVED BY THE INVENTION

In the light of the above situation, the problems to be solved by the present invention is to provide an analysis method that can sufficiently control the adsorbability considered to derive from the physical structure of a spherical carbon adsorbent and the like, and accurately evaluate a spherical carbon adsorbent, particularly, Kremezin (registered trade mark).

### MEANS OF SOLVING THE PROBLEMS

The present inventors have conducted intensive studies in an attempt to solve the aforementioned problems and found an adsorption test method capable of accurate evaluation of a spherical carbon adsorbent, particularly Kremezin (registered trade mark), based on the adsorbability of spherical carbon adsorbent, i.e., adsorption titer, adsorption speed and/or adsorption selectivity, by using, as a substance to be adsorbed, a compound satisfying certain conditions relating to the adsorbability of spherical carbon adsorbent for various substances, which resulted in the completion of the present invention.

Accordingly, the present invention provides the following. [1] A method of testing an adsorption selectivity of a spherical carbon adsorbent, comprising using a test solution simultaneously comprising as substances to be adsorbed, four kinds of compounds having the common chemical structure as that of the particular uremic toxic substance, and the four kinds of the compounds are (1) a compound having a hydrophobic group,
(2) a compound having a hydrophilic group,
(3) a compound having an acidic group and
(4) a compound having a basic group.

The test method of the above-mentioned [1], wherein the particular uremic toxic substance is indole, and the four kinds of compounds having the common chemical structure are (1) indole as a compound having a hydrophobic group,
(2) tryptophan as a compound having a hydrophilic group,
(3) indoleacetic acid as a compound having an acidic group, and
(4) tryptamine as a compound having a basic group.

The test method of the above-mentioned [1] or [2], wherein the test solution further comprises, as a substance to be adsorbed, a macromolecular compound having a molecular weight of 1000 - 1500.

The test method of the above-mentioned [3], wherein the macromolecular compound is cyanocobalamin.

The test method of any of the above-mentioned [1] to [4], comprising a step of adding a spherical carbon adsorbent to be a test substance to a test solution comprising a substance to be adsorbed, and allowing adsorption at a predetermined temperature for a predetermined time with stirring, and a step of measuring the concentration or amount of each substance to be adsorbed remaining in the test solution without the spherical carbon adsorbent.

The test method of the above-mentioned [5], wherein the concentration of each substance to be adsorbed in the test solution before addition of the spherical carbon adsorbent is 0.05 - 0.5 mg/mL.

The test method of the above-mentioned [6], wherein the concentration of each substance to be adsorbed in the test solution before addition of the spherical carbon adsorbent is 0.05 - 0.2 mg/mL.

The test method of the above-mentioned [7], wherein the concentration of each substance to be adsorbed in the test solution before addition of the spherical carbon adsorbent is 5 0.1 mg/mL.

The test method of any of the above-mentioned [5] to [8], wherein the test solution is obtained by dissolving each substance to be adsorbed in phosphate buffer at pH 6 - 8.

The test method of the above-mentioned [9], wherein the 10 spherical carbon adsorbent is added in an amount of 10 - 300 mg per 50 mL of the test solution.

The test method of the above-mentioned [10], wherein the spherical carbon adsorbent is added in an amount of 40 - 120 mg per 50 mL of the test solution.

The test method of the above-mentioned [11], wherein the spherical carbon adsorbent is added in an amount of 50 mg or 100 mg per 50 mL of the test solution.

The test method of any of the above-mentioned [10] to [12], wherein the volume of the test solution is 10 - 300 mL.

The test method of the above-mentioned [13], wherein the volume of the test solution is 30 - 100 mL.

The test method of the above-mentioned [14], wherein the volume of the test solution is 50 mL.

The test method of any of [5] to [15], wherein the 25 adsorption time is 1 - 6 hr, and the temperature is 10 - 50°C.

### EFFECT OF THE INVENTION

Using the analysis method of the present invention, the adsorbability of a spherical carbon adsorbent considered to derive from the physical structure and the like of the spherical carbon adsorbent can be accurately evaluated. Using the analysis method of the present invention, a pharmaceutical product comprising a spherical carbon adsorbent as an active ingredient, and a spherical carbon adsorbent, which is therapeutically and/or biologically equivalent thereto, can be accurately evaluated. Using the analysis method of the present invention, Kremezin (registered trade mark) comprising a spherical carbon adsorbent as an active ingredient can be accurately evaluated.

### BRIEF DESCRIPTION OF THE DRAWINGS

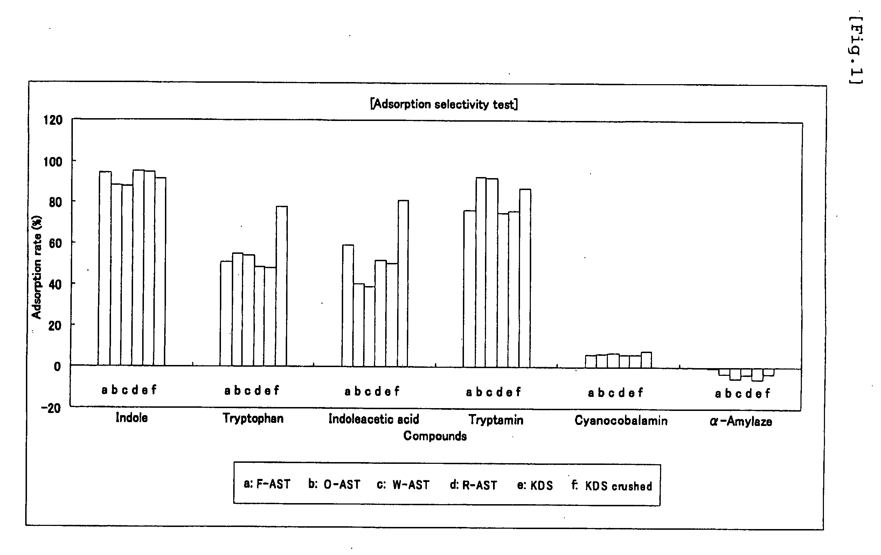

Fig. 1 shows adsorption selectivity of a spherical carbon adsorbent before an oxidization reduction treatment (F-AST), a spherical carbon adsorbent after an oxidation treatment (O-AST), a spherical carbon adsorbent after washing with water (W-AST), a spherical carbon adsorbent after a reduction treatment (R-AST), a spherical carbon adsorbent to be a bulk pharmaceutical (KDS) and a spherical carbon adsorbent to be a bulk pharmaceutical (KDS) after crushing (KDS crushed). As substances to be adsorbed, six kinds of compounds (indole, tryptophan, indoleacetic acid, tryptamine, cyanocobalamin and α-amylase) were used. Respective columns show adsorption rates of the above-mentioned 6 compounds to each spherical carbon adsorbent, wherein the adsorption rates of the compounds are arranged from the left in the order of a: F-AST, b: O-AST, c: W-AST, d: R-AST, e: KDS and f: KDS crushed.

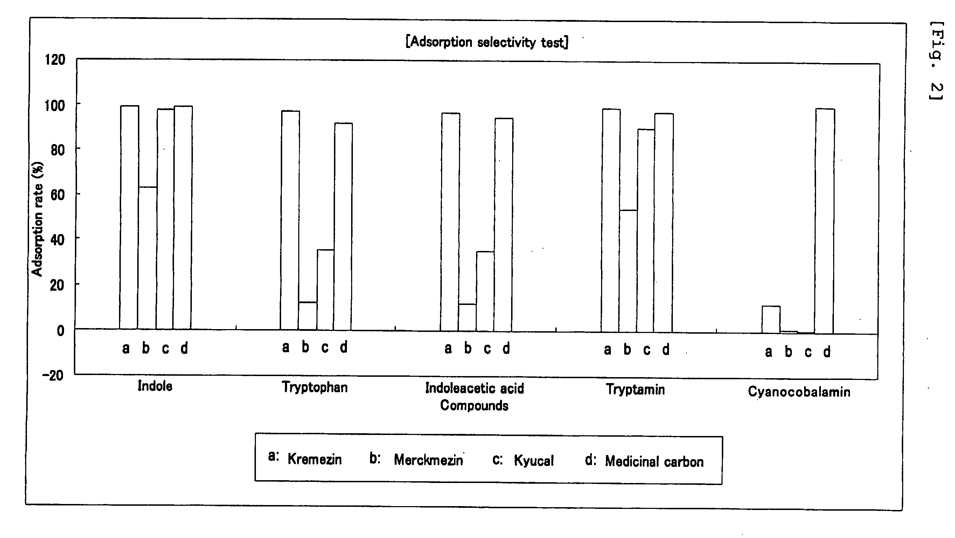

Fig. 2 shows adsorption selectivity to Kremezin (registered trade mark), Merckmezin (registered trade mark), Kyucal (registered trade mark) and medicinal carbon. As substances to be adsorbed, 5 compounds of indole, tryptophan, indoleacetic acid, tryptamine and cyanocobalamin were used. Respective columns show adsorption rates of the above-mentioned 5 compounds to each spherical carbon adsorbent, wherein the adsorption rates of the compounds are arranged from the left in the order of a: Kremezin, b: Merckmezin, c: Kyucal and d: medicinal carbon.

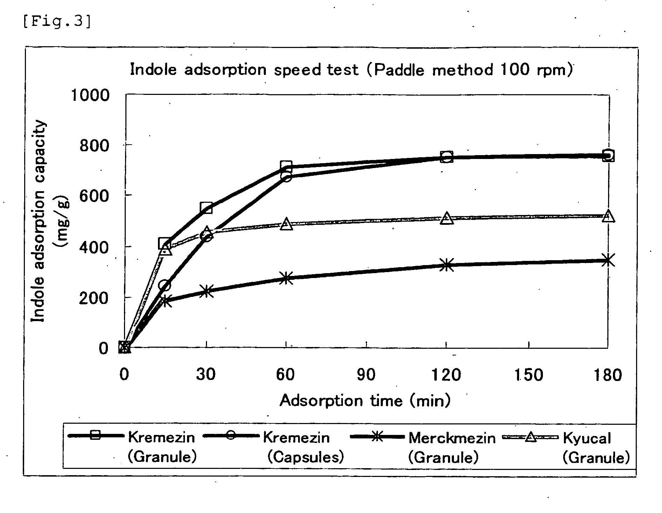

Fig. 3 shows the results of adsorption speed test of indole to Kremezin (registered trade mark), Kremezin (registered trade mark) capsule, Merckmezin (registered trade mark) and Kyucal (registered trade mark). The vertical axis of the graph shows adsorption amount of indole per 1 g of each spherical carbon adsorbent, and the horizontal axis shows adsorption time.

### EMBODIMENT OF INVENTION

The present invention is explained in detail in the following. For the evaluation of adsorbability of a spherical carbon adsorbent, evaluation of adsorbability of the spherical carbon adsorbent, namely, adsorption titer, adsorption rate and/or adsorption selectivity, is important. Therefore, the present invention provides an adsorption test method characterized by use of a test solution containing one or more kinds of particular uremic toxic substances or related compounds thereof. Specifically, it comprises a step of adding a spherical carbon adsorbent to be a test substance to the test solution, and allowing adsorption at a predetermined temperature for a predetermined time with stirring, a step of removing the spherical carbon adsorbent, and a step of measuring the concentration or amount of the substance to be adsorbed remaining in the test solution. By the adsorption test method, adsorbability of a spherical carbon adsorbent can be evaluated. The adsorption test method of the present invention comprises at least one test method selected from the group consisting of an adsorption titer test method, an adsorption speed test method and an adsorption selectivity test method.

The "spherical carbon adsorbent" to be used in the present specification is a spherical fine particulate porous carbon. For example, the spherical fine particulate porous carbon includes spherical carbon adsorbents for oral administration such as "Kremezin (registered trade mark)", "Merckmezin (registered trade mark)", "Kyucal (registered trade mark)" and the like. Preferred is "Kremezin (registered trade mark)". Furthermore, the "spherical carbon adsorbent" to be used in the present specification also includes "porous spherical carbonaceous substance" described in JP-B-3522708, "spherical activated carbon" and "surface-modified spherical activated carbon" described in each of JP-B-3672200, EP-A-1547605, JP-B- 3865400, EP-A-1745793, JP-B-3835698, EP-A-1500397, JP-B-3865399, EP-A-1500397 and JP-A-2000-233916, and "adsorbent for pharmaceutical agent" described in JP-A-2004-244414. These spherical carbon adsorbents can also be evaluated by the method of the present invention. However, the spherical carbon adsorbent is not limited to these. "Kremezin (registered trade mark)" is a spherical carbon adsorbent having a diameter of 0.01 - 1 mm, a specific surface area as calculated by the BET Theory of not less than 700 m²/g, a volume of pores having a pore diameter of 20 - 15000 nm ranges from not less than 0.04 mL/g to less than 0.10 mL/g, a total amount of acidic groups of 0.3 - 1.20 meq/g, and a total amount of basic groups of 0.20 - 0.70 meq/g.

### <Compounds to be used for the evaluation in the present invention>

(1) For evaluation of the adsorption titer, use of a target compound relating to the efficacy on chronic renal failure, and permitting measurement of the adsorption amount to a spherical carbon adsorbent to be evaluated by the present invention upon adsorption equilibrium (or adsorption saturation) thereof is important to ensure robust test conditions and quantification precision.

Thus, examples of the compound to be used for the evaluation of the above-mentioned adsorption titer include uremic toxin and related compounds thereof. Specific examples include the uremic toxic compounds described in Vanholder et al., Pediatric Nephrology, 2008, Vol. 23, p. 1211-1221, and EUToX Uremic Toxin Database (http://www.nephro-leipzig.de/eutoxdb/index.php), precursors thereof, metabolites thereof and the like. Furthermore, DL-β-aminoisobutyric acid, indole, p-cresol, advanced glycation end products (AGEs) and the like are also included, which have been evaluated as the compounds to be adsorbed by Kremezin. Among these compounds, preferred is a compound showing an adsorption amount to a spherical carbon adsorbent of 20 - 80%, and requiring not more than 24 hr to reach adsorption equilibrium (or adsorption saturation), and more preferred is a compound showing an adsorption amount to Kremezin (registered trade mark) of 20 - 80%, and requiring not more than 24 hr to reach adsorption equilibrium (or adsorption saturation).

Here, the "adsorption amount to a spherical carbon adsorbent of 20 - 80%, and requiring not more than 24 hr to reach adsorption equilibrium (or adsorption saturation)" can be evaluated according to the following method. The measurement time points are set to not less than 3 points including 3 hr and 24 hr (e.g., 3, 6, 24 and 48 hr and the like), and the test is finished at the time point when adsorption equilibrium (or adsorption saturation) was confirmed in any test system of spherical carbon adsorbent.

The adsorption equilibrium (or adsorption saturation) can be acknowledged when a residual ratio of the substance to be adsorbed is 20 - 80% and is a predetermined value (within error ±3% relative to the residual ratio at 24 hr).

In this test method, for example, it is desirable to prepare a test solution containing about 0.1 mg/mL of a substance to be adsorbed in phosphate buffer at pH 7.4, add 10 mg, 125 mg or 500 mg of a spherical carbon adsorbent to 50 mL of the test solution, and evaluate the adsorption amount at each time point in each test system.

Moreover, it is necessary for the compound to have sufficient stability under the above-mentioned measurement conditions. Particularly, the compound is not particularly limited as long as it satisfies the above-mentioned conditions, and preferable examples thereof include indole, p-cresol, hippuric acid, guanidinosuccinic acid, phenol and the like, with further preference given to indole.

The adsorption amount of DL-β-aminoisobutyric acid to Kremezin (registered trade mark) is extremely low as compared to the above-mentioned preferable compounds. In a test system using 500 mg of Kremezin, the adsorption amount reached 20 - 80%, though below adsorption equilibrium.

(2) For evaluation of adsorption speed, the compounds usable for the above-mentioned evaluation of adsorption titer can be preferably used.

(3) In evaluation of adsorption selectivity, adsorbability varies depending on the pore diameter and volume thereof, and modified surface condition of carbon by oxidation reduction treatment and the like, since the carbon adsorbent is spherical, fine particulate and porous. Therefore, the adsorbability of a spherical carbon adsorbent can be accurately evaluated by evaluating the selectivity of a compound to be used for this test as a substance to be adsorbed, based on the "molecular size", and factors such as "hydrophilicity, hydrophobicity, acidity and basicity" and the like.

In the present invention, it is preferable to use a combination of a compound to be used for the evaluation of the above-mentioned adsorption titer as a core compound and, as substances to be adsorbed, the core compound or substance(s) having the same chemical structure as that of the core compound, into which any one of the "hydrophilic, hydrophobic, acidic and basic" substituents has been introduced, namely, a compound having an acidic group introduced into said same chemical structure (hereinafter sometimes to be referred to as a "compound having an acidic group"), a compound having a basic group introduced into said same chemical structure (hereinafter sometimes to be referred to as a "compound having a basic group), a compound having a hydrophobic group introduced into said same chemical structure (including a core compound showing hydrophobicity, hereinafter sometimes to be referred to as a "compound having a hydrophobic group"), or a compound having a hydrophilic group introduced into said same chemical structure (hereinafter sometimes to be referred to as a "compound having a hydrophilic group").

Examples of the hydrophilic group include amino acid group, hydroxyl group and the like, and examples of the hydrophobic group include an alkyl group, a phenyl group and the like. Examples of the acidic group include a carboxyl group, a phosphoric acid group, a sulfuric acid group and the like, and examples of the basic group include an amino group, a guanidyl group and the like. The core compound or a substance having the same chemical structure as that of the core compound, into which any one of the above-mentioned substituents has been introduced, is sufficient as long as it is improved in the property corresponding to the introduced substituent (e.g., acidity, basicity, hydrophilicity, hydrophobicity and the like), as compared to the core compound or a substance having the same chemical structure as that of the core compound.

Here, the selected core compound can be directly used as a substance to be adsorbed in the test, without introduction of a substituent in consideration of the property thereof (e.g., acidity, basicity, hydrophilicity, hydrophobicity). For example, when indole showing hydrophobicity is selected as a core compound in the evaluation of the adsorption selectivity in the present invention, indole as a compound having a hydrophobic group, tryptophan having an amino acid group introduced into indole as a compound having a hydrophilic group, indoleacetic acid having a carboxyl group introduced into indole and/or indoxylsulfuric acid having a sulfuric acid group introduced into indole as a compound having an acidic group, and tryptamine having an amino group introduced into indole as a compound having a basic group can be used in combination as substances to be adsorbed in an adsorption selectivity test.

Preferred is a combination of four kinds of compounds of indole, tryptophan, indoleacetic acid and tryptamine, and they can be used for the adsorption selectivity test.

When a combination of selected substances to be adsorbed is used for the test, such substances to be adsorbed are dissolved in the same solution, and the solution is brought into contact with a spherical carbon adsorbent, based on which the adsorption selectivity of the spherical carbon adsorbent can be evaluated.

Moreover, the test can be performed in the co-presence of a non-adsorption substance besides the above-mentioned substance to be adsorbed. For example, when Kremezin (registered trade mark) is employed as a spherical carbon adsorbent for the test, since the molecule size-exclusion region of Kremezin is assumed to be a molecular weight of about 1000 - 1500, a compound having a molecular weight of 1000 - 1500, which is not adsorbed to Kremezin, is beneficially used.

While the non-adsorption substance here is not particularly limited, a compound present in the body is preferable, and cyanocobalamin (molecular weight 1355, hereinafter sometimes to be referred to as vitamin B₁₂) is particularly preferable.

α-Amylase is a protein having a molecular weight of 50000, and has conventionally been used as a non-adsorption substance in Kremezin adsorption tests. However, it has been clarified that α-amylase cannot be used as a non-adsorption substance in the present evaluation method in which plural substances to be adsorbed are simultaneously contained, since addition of α-amylase results in precipitation and turbidity.

### <Measurement methods for evaluation in the present invention>

### (1) Evaluation of adsorption titer

The adsorption titer can be evaluated according to a conventional method. For example, it comprises adding a spherical carbon adsorbent to be a test substance to a test solution comprising a substance to be adsorbed, and allowing adsorption at a predetermined temperature for a predetermined time with stirring, and measuring the concentration or amount of the substance to be adsorbed remaining in the test solution without the spherical carbon adsorbent.

The conditions of the concentration of a substance to be adsorbed, amount of the spherical carbon adsorbent to be evaluated, adsorption time, pH of solution, temperature and the like can be appropriately selected according to the kind of the substance to be adsorbed.

To be specific, for example, the following conditions can be nonlimitatively recited.

The concentration of the substance to be adsorbed is 0.1 - 10 mg/mL, preferably 0.5 - 1.5 mg/mL, more preferably 1.0 mg/mL.

While the amount of the test solution may be any as long as it permits measurement, it is 10 - 300 mL, preferably 30 - 100 mL, more preferably 50 mL.

The pH of the test solution is pH 6 - 8, preferably pH 6.8 or pH 7.4.

The test solution may be any as long as it is generally used, and a buffer is normally used. Preferred are acetate buffer, phosphate buffer, citrate buffer, borate buffer, tartrate buffer and the like, and more preferred is phosphate buffer.

The amount of the spherical carbon adsorbent to be evaluated can be appropriately determined according to the concentration of the substance to be adsorbed, volume of test solution, pH and/or kind of the buffer. For example, when the concentration of the substance to be adsorbed is 0.5 - 1.5 mg/mL and the volume of the test solution is 50 mL, the amount of the spherical carbon adsorbent to be added is 10 - 50 mg, preferably 10 - 30 mg, more preferably 25 mg.

The adsorption time can be appropriately determined according to the concentration of the substance to be adsorbed, volume of the test solution, pH, the kind of the buffer and/or the amount of the spherical carbon adsorbent to be evaluated. For example, it is 1 - 6 hr, preferably 3 hr.

The adsorption temperature is 10 - 50°C, preferably 30 - 40°C, more preferably 37°C.

When indole is used as a substance to be adsorbed, the adsorption amount does not change within the range of pH 2 - 12. However, as the adsorption temperature increases from 10°C to 50°C, the adsorption amount tends to decrease. Therefore, the adsorption titer test can be performed under the conditions of the concentration of indole in a test solution of 0.5 - 1.5 mg/mL, preferably 1 mg/ml, water or a buffer at pH 6 - 8, preferably phosphate buffer at pH 7.4, as a dissolution solution for a test solution, the amount of a spherical carbon adsorbent to be added of 10 - 30 mg, preferably 25 mg, relative to 50 mL of a test solution, adsorption temperature of 30 - 40°C, preferably 37°C, the volume of a test solution of 50 ml, and the adsorption time of 1 - 6 hr, preferably 3 hr after stirring in tumbler rotary stirrer ROTAR MIX 60 rpm.

In addition, the adsorption amount can be measured by a conventional method, for example, HPLC and the like.

For example, when the substance to be adsorbed is indole, the adsorption amount thereof can be measured by HPLC under the following conditions.

### HPLC conditions

Detector: ultraviolet absorption spectrophotometer (measurement wavelength: 240 nm)
Column: GL-Science Inertsil ODS-3V (4.6 mmID, 150 mm, 5 µm) or SUPELCO Discovery HS C18 (4.6 mmID, 150 mm, 5 µm)
Column temperature: 40°C
Mobile phase: water/acetonitrile/TFA mixture (1000:1000:1)
Flow: 1 mL/min
Injection volume: 10 µL

### (2) Evaluation of adsorption speed

The evaluation method of the adsorption speed comprises, for example, adding a spherical carbon adsorbent to be a test substance to a test solution comprising a substance to be adsorbed, and allowing adsorption at a predetermined temperature for a predetermined time with stirring, and measuring the concentration or amount of the substance to be adsorbed remaining in the test solution without the spherical carbon adsorbent.

In addition, the adsorption speed can be evaluated by, for example, a method in accordance with the dissolution test, 50 rpm or 100 rpm, of the Japanese Pharmacopoeia Paddle Method and/or the Japanese Pharmacopoeia Basket Method, 100 rpm, and a test solution containing a given concentration of a substance to be adsorbed can be used.

The conditions such as the concentration of a substance to be adsorbed, the amount of a spherical carbon adsorbent to be evaluated, pH of the solution and the like can be appropriately determined according to the kind and the like of the substance to be adsorbed.

To be specific, for example, the following conditions can be nonlimitatively recited.

The concentration of the substance to be adsorbed is 0.1 - 10 mg/mL, preferably 0.5 - 1.5 mg/mL, more preferably 1.0 mg/mL.

While the amount of the test solution containing a substance to be adsorbed may be any as long as it permits measurement, it is 10 mL - 3 L, preferably 100 mL - 2 L, more preferably 900 mL.

The pH of the test solution is pH 6 - 8, preferably pH 6.8 or pH 7.4.

The test solution may be any as long as it is a buffer generally used. Preferred are acetate buffer, phosphate buffer, citrate buffer, borate buffer, tartrate buffer and the like, and more preferred is phosphate buffer.

The amount of the spherical carbon adsorbent to be evaluated can be appropriately determined according to the concentration of the substance to be adsorbed, volume of test solution, pH and/or kind of the buffer. For example, when the concentration of the substance to be adsorbed is 0.5 - 1.5 mg/mL and the volume of the test solution is 900 mL, the amount of the spherical carbon adsorbent to be added is 100 - 600 mg, preferably 180 - 540 mg, more preferably 300 mg.

In the evaluation method of the adsorption speed in the present invention, the adsorption time can be appropriately determined according to the concentration of the substance to be adsorbed, volume of the test solution, pH, the kind of the buffer and/or the amount of the spherical carbon adsorbent to be evaluated. For example, it is 15 min - 6 hr.

In the evaluation method of the adsorption speed in the present invention, the adsorption temperature is 10 - 50°C, preferably 30 - 40°C, more preferably 37°C.

### (3) Evaluation of adsorption selectivity

The adsorption selectivity can be evaluated by a conventional method. The method comprises, for example, adding a spherical carbon adsorbent to be a test substance to a test solution comprising substances to be adsorbed, and allowing adsorption at a predetermined temperature for a predetermined time with stirring, and measuring the concentration or amount of each substance to be adsorbed remaining in the test solution without the spherical carbon adsorbent.

When a particular uremic toxin substance is a core compound, the substance to be adsorbed here comprises a group of four kinds of substances to be adsorbed, consisting of a compound having an acidic group introduced into the same chemical structure as that of the core compound (hereinafter sometimes to be referred to as a "compound having an acidic group"), a compound having a basic group introduced into said same chemical structure (hereinafter sometimes to be referred to as a "compound having a basic group), a compound having a hydrophobic group introduced into said same chemical structure (hereinafter sometimes to be referred to as a "compound having a hydrophobic group"), or a compound having a hydrophilic group introduced into said same chemical structure (hereinafter sometimes to be referred to as a "compound having a hydrophilic group"). In some cases, a macromolecular compound having a molecular weight of 1000 - 1500 can also be used as a non-adsorption substance in addition to the above-mentioned group of substances to be adsorbed.

The conditions of the concentration of each substance to be adsorbed, amount of the spherical carbon adsorbent to be evaluated, adsorption time, pH of test solution, temperature and the like need to be appropriately selected according to the kind of the substances to be adsorbed.

Particularly, in this evaluation method, the kind of the substances to be adsorbed influences the adsorption amount of the substances to be adsorbed. However, the adsorption selectivity can be evaluated by maintaining the constant conditions of the concentration of substances to be adsorbed, amount of spherical carbon adsorbent to be evaluated, adsorption time, pH of test solution, temperature and the like.

To be specific, for example, the following conditions can be nonlimitatively recited.

The concentration of each of the four kinds of substances to be adsorbed consisting of a compound having an acidic group, a compound having a basic group, a compound having a hydrophobic group, and a compound having a hydrophilic group, each of which is based on a particular uremic toxic substance as a core compound, or five kinds consisting of the above four compounds and a macromolecular compound having a molecular weight of 1000 - 1500 is 0.05 - 0.5 mg/mL, preferably 0.05 - 0.2 mg/mL, more preferably 0.1 mg/mL.

While the amount of the test solution containing the above-mentioned four kinds of compounds, or five kinds including the macromolecular compound, may be any as long as it permits measurement, it is 10 - 300 mL, preferably 30 - 100 mL, more preferably 50 mL.

The pH of the test solution is pH 6 - 8, preferably pH 6.8 or pH 7.4.

The test solution may be any as long as it is a buffer generally used. Preferred are acetate buffer, phosphate buffer, citrate buffer, borate buffer, tartrate buffer and the like, and more preferred is phosphate buffer.

The amount of the spherical carbon adsorbent to be evaluated can be appropriately determined according to the concentration of the substance to be adsorbed, volume of test solution, pH and/or kind of the buffer. For example, when the concentration of the substance to be adsorbed is 0.05 - 0.5 mg/mL and the volume of the test solution is 50 mL, the amount of the spherical carbon adsorbent to be added is 10 - 300 mg, preferably 40 - 120 mg, more preferably 50 - 100 mg, still more preferably 50 mg or 100 mg.

The adsorption time can be appropriately determined according to the concentration of the substance to be adsorbed, volume of the test solution, pH, the kind of the buffer and/or the amount of the spherical carbon adsorbent to be evaluated. For example, it is 1 - 6 hr, preferably 3 hr.

The adsorption temperature is 10 - 50°C, preferably 30 - 40°C, more preferably 37°C.

It is preferable to perform evaluation under adsorption pH and adsorption temperature conditions in which the spherical carbon adsorbent generally exhibits its effects in the body.

When the substances to be adsorbed are 4 kinds of indole (compound having a hydrophobic group), tryptophan (compound having a hydrophilic group), indoleacetic acid (compound having an acidic group) and tryptamine (compound having a basic group), and the non-adsorption substance is cyanocobalamin, the adsorption selectivity of each substance to be adsorbed can be evaluated under the conditions of the concentration of each substance in a test solution: 0.05 - 0.5 mg/mL, preferably 0.05 - 0.2 mg/mL, more preferably 0.1 mg/mL or 0.2 mg/mL, dissolution solution for a test solution: water or a buffer at pH 6 - 8, preferably phosphate buffer at pH 7.4, the amount of a spherical carbon adsorbent to be added: 10 - 300 mg, preferably 40 - 120 mg, more preferably 50 - 100 mg, still more preferably 50 mg or 100 mg, relative to 50 mL of a test solution, adsorption temperature: 10 - 50°C, preferably 30 - 40°C, more preferably 37°C, the volume of a test solution: 50 ml, and the adsorption time: 1 - 6 hr, preferably 3 hr after stirring in tumbler rotary stirrer ROTAR MIX 60 rpm.

In addition, the adsorption amount can be measured by a conventional method, for example, HPLC and the like.

The adsorption amount of each substance to be adsorbed can be measured by HPLC under the following conditions.
Detector: ultraviolet absorption spectrophotometer (measurement wavelength: 240 nm)
Column: GL-Science Inertsil ODS-3V (4.6 mmID, 150 mm, 5 µm) or SUPELCO Discovery HS C18 (4.6 mmID, 150 mm, 5 µm)
Column temperature: 40°C
Mobile phase A: water/TFA mixture (2000:1)
Mobile phase B: acetonitrile/TFA mixture (2000:1)
Solution sending conditions: gradient analysis conditions Mobile phase B concentration 5%→80% (30 min)
Flow: 1 mL/min
Injection volume: 10 µL

### EXAMPLES

The present invention is explained in more detail in the following by referring to Examples, which are not to be construed as limitative.

### [Example 1: Evaluation test of adsorption titer]

### (Test method)

Sample: A porous spherical carbonaceous substance produced according to the production method described in Example 1 of JP-B-3522708 was used as a spherical carbon adsorbent to be the test substance (Sample-1) for this test.

Evaluation test of adsorption titer: The spherical carbon adsorbent (25 mg) was added to a phosphate buffer containing indole (pH 7.4, concentration 1.0 mg/mL, 50 mL), the mixture was shaken at 37°C for 3 hr, and the residual concentration of indole was measured by HPLC.

As a stirrer, a tumbler rotary stirrer (ROTAR MIX) was used at a stirring rate of 60 rpm.

The HPLC conditions were as follows.
Detector: ultraviolet absorption spectrophotometer (measurement wavelength: 240 nm)
Column: GL-Science Inertsil ODS-3V (4.6 mmID, 150 mm, 5 µm)
Column temperature: 40°C
Mobile phase: water/acetonitrile/TFA mixture (1000:1000:1)
Flow: 1 mL/min
Injection volume: 10 µL

The amount of indole adsorbed per 1 g of the spherical carbon adsorbent in each lot was calculated from the difference in the concentration before and after the adsorption, and compared as an adsorption titer for evaluation. The test results are shown in Table 1.

**Table 1: adsorption titer**

| Lot number | Indole adsorption amount (mg/g)*¹ |
|---|---|
| 1 | 696 |
| 2 | 711 |
| 3 | 690 |
| 4 | 686 |
| 5 | 705 |
| 6 | 699 |
| 7 | 695 |

| | |
|---|---|
| *1 average of 3 repeats | |

### (Test results)

Evaluation of adsorption titer: The adsorption amount of indole per 1 g of the spherical carbon adsorbent (Sample-1) of 7 lots used in this test was a constant amount of 700 mg/g ± 10%.

### [Example 2: Evaluation test of adsorption titer]

### (Test method)

Sample: From among the porous spherical carbonaceous substances produced according to the production method described in Example 1 of JP-B-3522708, spherical carbon adsorbent before oxidation and reduction treatment (F-AST), spherical carbon adsorbent after oxidation treatment (O-AST), spherical carbon adsorbent after washing with water (W-AST), spherical carbon adsorbent after reduction treatment (R-AST), spherical carbon adsorbent to be a bulk pharmaceutical (KDS), and crushed spherical carbon adsorbent to be a bulk pharmaceutical (KDS) (KDS crushed) were used as the spherical carbon adsorbents.

The evaluation test of adsorption titer was performed in the same manner as in Example 1. The adsorption temperature was room temperature. The test results are shown in Table 2.

**Table 2: test results of various spherical carbon adsorbents and crushed product thereof**

| Test substance | Indole adsorption amount (mg/g) |
|---|---|
| F-AST | 704.9 |
| O-AST | 696.9 |
| W-AST | 659.9 |
| R-AST | 679.9 |
| KDS | 679.1 |
| KDS crushed | 675.5 |

### (Test results)

Evaluation of adsorption titer: The spherical carbon adsorbent before oxidation and reduction treatment (F-AST), spherical carbon adsorbent after oxidation treatment (O-AST), spherical carbon adsorbent after washing with water (W-AST), spherical carbon adsorbent after reduction treatment (R-AST), spherical carbon adsorbent to be a bulk pharmaceutical (KDS) and crushed spherical carbon adsorbent to be a bulk pharmaceutical (KDS) (KDS crushed) adsorbed indole well, and the adsorption amount of the spherical carbon adsorbents in each treatment step was a constant amount of 700 mg/g ± 10%. Therefrom it is suggested that the adsorption titer property of the spherical carbon adsorbent relative to indole is not influenced by an oxidation/reduction treatment step and the like. Therefore, the adsorption titer property of the spherical carbon adsorbent is considered to be determined by the production step before the oxidation/reduction treatment step and the quality of the spherical carbon adsorbent.

### [Example 3: Evaluation test of adsorption titer]

### (Test method)

Sample: Kremezin (registered trade mark) fine granules (manufactured by Kureha Chemical Industry Co., Ltd., production number 74K2) (Kre) produced according to the production method described in Example 1 of JP-B-3522708, Merckmezin (registered trade mark) fine granules (manufactured by MERCK & CO., INC., production number 038OMK) (Mer) and Kyucal (registered trade mark) fine granules (manufactured by Teikoku Medix, production number 6EB) (Kyu) were used as spherical carbon adsorbents. In addition, a medicinal carbon (manufactured by ORIENTAL PHARMACEUTICAL Co., Ltd., production number 70525) (Yak), which is an adsorbent for oral administration, was used. Merckmezin fine granules (Mer), Kyucal fine granules (Kyu) and medicinal carbon (Yak) used for this test were purchased. The evaluation test of adsorption titer was performed in the same manner as in Example 1. The test results are shown in Table 3.

**Table 3: comparison of indole adsorption amounts of Kremezin and competitive drugs**

| Sample name | Indole adsorption amount (mg/g) |
|---|---|
| Kre | 662 |
| Mer | 319 |
| Kyu | 523 |
| Yak | 387 |

### (Test results)

Evaluation of adsorption titer: The amount of indole adsorbed per 1 g of the spherical carbon adsorbent decreased in the order of Kre (662 mg/g), Kyu (523 mg/g) and Mer (319 mg/g). Yak adsorbed 387 mg/g of indole.

### [Example 4: Evaluation test of adsorption selectivity]

### (Test method)

Sample: A spherical carbon adsorbent (Sample-2) produced according to the production method described in Example 1 of JP-B-3522708 was used as a spherical carbon adsorbent.

### Evaluation method of adsorption selectivity:

A spherical carbon adsorbent (100 mg) was added to a phosphate buffer containing five compounds of indole, tryptophan, indoleacetic acid, tryptamine and cyanocobalamin (pH 7.4, concentration of each compound; 0.1 mg/mL, 50 mL), the mixture was shaken at 37°C for 3 hr, and the residual concentration of each compound in the test solution was measured by HPLC. As a stirrer, a tumbler rotary stirrer (ROTAR MIX) was used at a stirring rate of 60 rpm.

The HPLC conditions are as follows.
Detector: ultraviolet absorption spectrophotometer (measurement wavelength: 240 nm)
Column: GL-Science Inertsil ODS-3V (4.6 mmID, 150 mm, 5 µm) Column temperature: 40°C
Mobile phase A: water/TFA mixture (2000:1)
Mobile phase B: acetonitrile/TFA mixture (2000:1)
Solution sending conditions: gradient analysis conditions Concentration of mobile phase B: 5→80% (30 min)
Flow: 1 mL/min
Injection volume: 10 µL

The adsorption rate of each compound to the spherical carbon adsorbent was calculated from the difference in the concentration between before and after adsorption, and the adsorption selectivity profile was compared for evaluation. The test results are shown in Table 4.

**Table 4: test results: concentration of each test solution 0.1 mg/mL, sample addition amount 100 mg**

| Lot number | Indole adsorption rate (%) | Tryptophan adsorption rate (%) | Indoleacetic acid adsorption rate (%) | Tryptamine adsorption rate (%) | Cyanocobalamin adsorption rate (%) |
|---|---|---|---|---|---|
| 1 | 99 | 97 | 98 | 99 | 11 |
| 2 | 99 | 98 | 98 | 99 | 7 |
| 3 | 99 | 96 | 97 | 99 | 10 |
| 4 | 99 | 92 | 93 | 99 | 6 |
| 5 | 99 | 98 | 98 | 99 | 6 |
| 6 | 99 | 97 | 98 | 99 | 8 |
| 7 | 99 | 97 | 97 | 99 | 6 |

### (Test results)

Evaluation of adsorption titer: The 7 lots of spherical carbon adsorbents (Sample-2) used at this time adsorbed indole, tryptophan, indoleacetic acid and tryptamine well, but did not adsorb cyanocobalamin. The adsorption rate (%) thereof was not less than 95% for indole, not less than 85% for tryptophan, not less than 85% for indoleacetic acid, and not less than 95% for tryptamine. However, only not less than 20% of cyanocobalamin was adsorbed. The adsorption amount of each lot to the spherical carbon adsorbent was almost the same.

### [Example 5: Evaluation test of adsorption selectivity]

### (Test method)

Sample: From among the porous spherical carbonaceous substances produced according to the production method described in Example 1 of JP-B-3522708, spherical carbon adsorbent before oxidation and reduction treatment (F-AST), spherical carbon adsorbent after oxidation treatment (O-AST), spherical carbon adsorbent after washing with water (W-AST), spherical carbon adsorbent after reduction treatment (R-AST), spherical carbon adsorbent to be a bulk pharmaceutical (KDS), and crushed spherical carbon adsorbent to be a bulk pharmaceutical (KDS) (KDS crushed) were used as the spherical carbon adsorbents. Evaluation method of adsorption selectivity:

Each spherical carbon adsorbent (50 mg) was added to a phosphate buffer containing six compounds of indole, tryptophan, indoleacetic acid, tryptamine, α-amylase and cyanocobalamin (pH 7.4, concentration of each compound; 0.1 mg/mL, 50 mL), the mixture was shaken at room temperature for 3 hr, and the residual concentration of each compound in the solution was measured by HPLC. As a stirrer, a tumbler rotary stirrer (ROTAR MIX) was used at a stirring rate of 60 rpm. Other conditions were the same as in Example 4. The test results are shown in Table 5 and Fig. 1.

**Table 5: test results: concentration of each test solution 0.1 mg/mL, sample addition amount 50 mg**

| Sample name | Indole adsorption rate (%) | Tryptophan adsorption rate (%) | Indoleacetic acid adsorption rate (%) | Tryptamine adsorption rate (%) | Cyanoco-balamin adsorption rate tion (%) | α-Amylase adsorption rate (%) |
|---|---|---|---|---|---|---|
| F-AST | 95 | 51 | 59 | 76 | 6 | 0 |
| O-AST | 88 | 55 | 41 | 93 | 7 | -3 |
| W-AST | 88 | 54 | 39 | 92 | 7 | -5 |
| R-AST | 95 | 48 | 52 | 75 | 6 | -3 |
| KDS | 95 | 48 | 50 | 76 | 6 | -5 |
| KDS crushed | 92 | 77 | 81 | 87 | 8 | -3 |

### (Test results)

Evaluation of adsorption selectivity: The spherical carbon adsorbent before oxidation and reduction treatment (F-AST), spherical carbon adsorbent after oxidation treatment (O-AST), spherical carbon adsorbent after washing with water (W-AST), spherical carbon adsorbent after reduction treatment (R-AST), spherical carbon adsorbent to be a bulk pharmaceutical (KDS) and crushed spherical carbon adsorbent to be a bulk pharmaceutical (KDS) (KDS crushed) tended to adsorb indole, tryptophan, indoleacetic acid and tryptamine well, and tended to not adsorb cyanocobalamin and α-amylase.

On the other hand, as for the adsorption amount of the substance to be adsorbed to the spherical carbon adsorbent before oxidation and reduction treatment (F-AST), spherical carbon adsorbent after oxidation treatment (O-AST), spherical carbon adsorbent after washing with water (W-AST), spherical carbon adsorbent after reduction treatment (R-AST), spherical carbon adsorbent to be a bulk pharmaceutical (KDS) and crushed spherical carbon adsorbent to be a bulk pharmaceutical (KDS) (KDS crushed), the adsorption amount of indole and indoleacetic acid to O-AST and W-AST decreased, whereas the adsorption amount thereof to R-AST after a reduction treatment, and KDS tended to increase.

On the other hand, the adsorption amount of tryptophan and tryptamine to O-AST and W-AST increased, and the adsorption amount thereof to R-AST after a reduction treatment, and KDS tended to increase.

As mentioned above, the method enables evaluation of the adsorbability based on the difference in the quality of spherical carbon adsorbents due to the level of oxidization or reduction treatment in the production process.

### [Example 6: Evaluation test of adsorption selectivity]

### (Test method)

Sample: As in Example 3, 4 kinds of spherical carbon adsorbents (Kre, Kyu, Mer and Yak) were used as spherical carbon adsorbents.

### Evaluation method of adsorption selectivity:

Spherical carbon adsorbent (100 mg) was added to a phosphate buffer containing five compounds of indole, tryptophan, indoleacetic acid, tryptamine and cyanocobalamin (pH 7.4, concentration of each compound; 0.1 mg/mL, 50 mL), the mixture was shaken at 37°C for 3 hr, and the residual concentration of each compound in the test solution was measured by HPLC. As a stirrer, a tumbler rotary stirrer (ROTAR MIX) was used at a stirring rate of 60 rpm. Other conditions were the same as those in Example 4. The test results are shown in Table 6 and Fig. 2.

**Table 6: adsorption selectivity test results: each test solution concentration 0.1 mg/mL, sample addition amount 100 mg**

| Sample name | Indole adsorption rate (%) | Tryptophan adsorption rate (%) | Indoleacetic acid adsorption rate (%) | Tryptamine adsorption rate (%) | Cyanocobalamin adsorption rate (%) |
|---|---|---|---|---|---|
| Kre | 99.0 | 97.4 | 97.1 | 99.1 | 11.9 |
| Mer | 63.0 | 12.3 | 12.2 | 54.0 | 0.8 |
| Kyu | 98.0 | 35.4 | 35.0 | 90.1 | 0.7 |
| Yak | 99.2 | 91.6 | 94.6 | 97.5 | 100.0 |

### (Test results)

Evaluation of adsorption selectivity: Kre adsorbed indole, tryptophan, indoleacetic acid and tryptamine well, and scarcely adsorbed cyanocobalamin. In contrast, Mer adsorbed indole and tryptamine to a certain level but scarcely adsorbed tryptophan, indoleacetic acid and cyanocobalamin. Kyu adsorbed indole and tryptamine well, somewhat adsorbed tryptophan and indoleacetic acid, and scarcely adsorbed cyanocobalamin. In addition, medicinal carbon did not show adsorption selectivity, and adsorbed all the compounds used for the test.

From the above results, the test method of the present invention is considered to be capable of clearly showing difference in the adsorbability of each spherical carbon adsorbent. Moreover, the test method of the present invention is considered to enable stable supply of high quality pharmaceutical products.

### [Example 7: Evaluation test of adsorption speed]

### (Test method)

Sample: As spherical carbon adsorbents, Kremezin (registered trade mark) fine granules (manufactured by Kureha Chemical Industry Co., Ltd., production number 74K2)(Kre), Kremezin (registered trade mark) capsule (manufactured by Kureha, production number 74K2)(Kre(capsel)), Merckmezin (registered trade mark) fine granules (manufactured by MERCK & CO., INC., production number 0380MK)(Mer) and Kyucal (registered trade mark) fine granules (manufactured by Teikoku Medix, production number 6EB) (Kyu) were used. The spherical carbon adsorbents used for the test were purchased.

### Evaluation method of adsorption speed:

The adsorption speed (in vitro biological equivalency test) was evaluated by the same method as in the dissolution test of the Japanese Pharmacopoeia Paddle Method, 100 rpm, used for the dissolution test of preparation and the like. The adsorption speed was calculated by dissolving indole (1.0 mg/mL) as a compound to be adsorbed in phosphate buffer at pH 7.4 to give a test solution (900 mL), adding a spherical carbon adsorbent (300 mg), and measuring the adsorption amount for a given time (15 min, 30 min, 60 min, 120 min, 180 min), i.e., indole adsorption capacity (IAC). The test results are shown in Table 7 and Fig. 3.

**Table 7: adsorption speed of Paddle Method 100 rpm**

| Adsorption time (min) | Paddle method 100 rpm | | | |
|---|---|---|---|---|
| | Kremezin (Kre) | Kremezin Capsel (Kre(Capsel)) | Merckmezin (Mer) | Kyucal (Kyu) |
| | IAC (mg/g) | IAC (mg/g) | IAC (mg/g) | IAC (mg/g) |
| 0 | 0 | 0 | 0 | 0 |
| 15 | 406 | 245 | 183 | 388 |
| 30 | 548 | 438 | 223 | 458 |
| 60 | 716 | 675 | 278 | 491 |
| 120 | 751 | 754 | 330 | 516 |
| 180 | 757 | 763 | 348 | 526 |

### (Test results)

Evaluation of adsorption speed: Kre (fine granules and capsule) adsorbed indole [406 mg/g and 245 mg/g for 15 min, 548 mg/g and 438 mg/g for 30 min, 716 mg/g and 675 mg/g for 60 min], and the adsorption amount of indole reached 700 mg/g ± 10% in not less than 60 min. On the other hand, Mer (fine granules) and Kyu (fine granules) adsorbed indole [183 mg/g and 385 mg/g for 15 min, 223 mg/g and 455 mg/g for 30 min, and 278 mg/g and 488 mg/g for 60 min], and thereafter, the adsorption amount reached equilibrium at about 300 mg/g and about 500 mg/g. Therefrom it is clear that Kre shows a similar adsorption rate irrespective of the dosage form. On the other hand, generic drugs, Mer (fine granules) and Kyu (fine granules), showed different values from Kre (fine granules and capsule) in the evaluation of adsorption speed.

### INDUSTRIAL APPLICABILITY

Spherical carbon adsorbents represented by Kremezin (registered trade mark) are produced under strict control of starting materials and production methods, and are physical pharmaceutical products having a physical structure considered to significantly influence adsorption of a toxic substance, and efficacy thereof.

The test method of the present invention can accurately evaluate adsorbability considered to derive from the physical structure of a spherical carbon adsorbent, particularly, Kremezin (registered trade mark), and can stably supply high quality pharmaceutical products. In addition, the equivalency relating to the adsorbability of generic drugs of Kremezin (registered trade mark) can be accurately evaluated simultaneously.

## Claims

1. A method of testing an adsorption selectivity of a spherical carbon adsorbent comprising using a test solution simultaneously comprising, as substances to be adsorbed, four kinds of compounds having the common chemical structure as that of the particular uremic toxic substance, and the four kinds of the compounds are
(1) a compound having a hydrophobic group,
(2) a compound having a hydrophilic group,
(3) a compound having an acidic group and
(4) a compound having a basic group.

2. The test method according to claim 1, wherein the particular uremic toxic substance is indole, and the four kinds of compounds having the common chemical structure are
(1) indole as a compound having a hydrophobic group,
(2) tryptophan as a compound having a hydrophilic group,
(3) indoleacetic acid as a compound having an acidic group, and
(4) tryptamine as a compound having a basic group.

3. The test method according to claim 1 or 2, wherein the test solution further comprises, as a substance to be adsorbed, a macromolecular compound having a molecular weight of 1000 - 1500.

4. The test method according to claim 3, wherein the macromolecular compound is cyanocobalamin.

5. The test method according to any one of claims 1 to 4, comprising a step of adding a spherical carbon adsorbent to be a test substance to a test solution comprising a substance to be adsorbed, and allowing adsorption at a predetermined temperature for a predetermined time with stirring, and a step of measuring the concentration or amount of each substance to be adsorbed remaining in the test solution without the spherical carbon adsorbent.

6. The test method according to claim 5, wherein the concentration of each substance to be adsorbed in the test solution before addition of the spherical carbon adsorbent is 0.05 - 0.5 mg/mL.

7. The test method according to claim 6, wherein the concentration of each substance to be adsorbed in the test solution before addition of the spherical carbon adsorbent is 0.05 - 0.2 mg/mL.

8. The test method according to claim 7, wherein the concentration of each substance to be adsorbed in the test solution before addition of the spherical carbon adsorbent is 0.1 mg/mL.

9. The test method according to any one of claims 5 to 8, wherein the test solution is obtained by dissolving each substance to be adsorbed in phosphate buffer at pH 6 - 8.

10. The test method according to claim 5, wherein the spherical carbon adsorbent is added in an amount of 10 - 300 mg per 50 mL of the test solution.

11. The test method according to claim 10, wherein the spherical carbon adsorbent is added in an amount of 40 - 120 mg per 50 mL of the test solution.

12. The test method according to claim 11, wherein the spherical carbon adsorbent is added in an amount of 50 mg or 100 mg per 50 mL of the test solution.

13. The test method according to any one of claims 10 to 12, wherein the volume of the test solution is 10 - 300 mL.

14. The test method according to claim 13, wherein the volume of the test solution is 30 - 100 mL.

15. The test method according to claim 14, wherein the volume of the test solution is 50 mL.

16. The test method according to any one of claims 5 to 15, wherein the adsorption time is 1 - 6 hr, and the temperature is 10 - 50°C.

## Patentansprüche

1. Verfahren zum Testen einer Adsorptionsselektivität eines kugelförmigen Kohlenstoffadsorbens, umfassend die Verwendung einer Testlösung, welche als zu adsorbierende Substanzen gleichzeitig vier Arten von Verbindungen umfasst, welche die gemeinsame chemische Struktur wie diejenige der besonderen urämischen toxischen Substanz aufweisen, wobei die vier Arten der Verbindungen sind
(1) eine Verbindung mit einer hydrophoben Gruppe,
(2) eine Verbindung mit einer hydrophilen Gruppe,
(3) eine Verbindung mit einer sauren Gruppe und
(4) eine Verbindung mit einer basischen Gruppe.

2. Testverfahren nach Anspruch 1, wobei die besondere urämische toxische Substanz Indol ist, und die vier Arten von Verbindungen mit der gemeinsamen chemischen Struktur sind
(1) Indol als eine Verbindung mit einer hydrophoben Gruppe,
(2) Tryptophan als eine Verbindung mit einer hydrophilen Gruppe,
(3) Indolessigsäure als eine Verbindung mit einer sauren Gruppe und
(4) Tryptamin als eine Verbindung mit einer basischen Gruppe.

3. Testverfahren nach Anspruch 1 oder 2, wobei die Testlösung weiterhin als eine zu adsorbierende Substanz eine makromolekulare Verbindung mit einem Molekulargewicht von 1000 - 1500 umfasst.

4. Testverfahren nach Anspruch 3, wobei die makromolekulare Verbindung Cyanocobalamin ist.

5. Testverfahren nach mindestens einem der Ansprüche 1 bis 4, umfassend einen Schritt des Zugebens eines kugelförmigen Kohlenstoffadsorbens als eine Testsubstanz zu einer Testlösung, welche eine zu adsorbierende Substanz umfasst, und Adsorbierenlassen bei einer vorbestimmten Temperatur während einer vorbestimmten Zeit unter Rühren, und einen Schritt des Messens der Konzentration oder Menge jeder zu adsorbierenden Substanz, welche in der Testlösung verbleibt, ohne das kugelförmige Kohlenstoffadsorbens.

6. Testverfahren nach Anspruch 5, wobei die Konzentration jeder zu adsorbierenden Substanz in der Testlösung vor Zugabe des kugelförmigen Kohlenstoffadsorbens 0,05 - 0,5 mg/ml beträgt.

7. Testverfahren nach Anspruch 6, wobei die Konzentration jeder zu adsorbierenden Substanz in der Testlösung vor Zugabe des kugelförmigen Kohlenstoffadsorbens 0,05 - 0,2 mg/ml beträgt.

8. Testverfahren nach Anspruch 7, wobei die Konzentration jeder zu adsorbierenden Substanz in der Testlösung vor Zugabe des kugelförmigen Kohlenstoffadsorbens 0,1 mg/ml beträgt.

9. Testverfahren nach mindestens einem der Ansprüche 5 bis 8, wobei die Testlösung erhalten wird durch Auflösen jeder zu adsorbierenden Substanz in Phosphatpuffer bei pH 6 -8.

10. Testverfahren nach Anspruch 5, wobei das kugelförmige Kohlenstoffadsorbens in einer Menge von 10 - 300 mg pro 50 ml der Testlösung zugegeben wird.

11. Testverfahren nach Anspruch 10, wobei das kugelförmige Kohlenstoffadsorbens in einer Menge von 40 - 120 mg pro 50 ml der Testlösung zugegeben wird.

12. Testverfahren nach Anspruch 11, wobei das kugelförmige Kohlenstoffadsorbens in einer Menge von 50 mg oder 100 mg pro 50 ml der Testlösung zugegeben wird.

13. Testverfahren nach mindestens einem der Ansprüche 10 bis 12, wobei das Volumen der Testlösung 10 - 300 ml beträgt.

14. Testverfahren nach Anspruch 13, wobei das Volumen der Testlösung 30 - 100 ml beträgt.

15. Testverfahren nach Anspruch 14, wobei das Volumen der Testlösung 50 ml beträgt.

16. Testverfahren nach mindestens einem der Ansprüche 5 bis 15, wobei die Adsorptionszeit 1 - 6 Stunden und die Temperatur 10 - 50°C beträgt.

## Revendications

1. Procédé de test de la sélectivité d'adsorption d'un adsorbant carboné sphérique comprenant l'utilisation d'une solution d'essai simultanément comprenant, en tant que substances à adsorber, quatre types de composés ayant la même structure chimique que celle de la substance toxique urémique particulière, et les quatre types de composés sont
(1) un composé comportant un groupe hydrophobe,
(2) un composé comportant un groupe hydrophile,
(3) un composé comportant un groupe acide et
(4) un composé comportant un groupe basique.

2. Procédé de test selon la revendication 1, dans lequel la substance toxique urémique particulière est l'indole, et les quatre types de composés ayant la structure chimique identique sont
(1) l'indole en tant que composé comportant un groupe hydrophobe,
(2) le tryptophane en tant que composé comportant un groupe hydrophile,
(3) l'acide indole-acétique en tant que composé comportant un groupe acide, et
(4) la tryptamine en tant que composé comportant un groupe basique.

3. Procédé de test selon la revendication 1 ou 2, dans lequel la solution d'essai comprend en outre, en tant que substance à adsorber, un composé macromoléculaire ayant un poids moléculaire de 1 000 à 1 500.

4. Procédé de test selon la revendication 3, dans lequel le composé macromoléculaire est la cyanocobalamine.

5. Procédé de test selon l'une quelconque des revendications 1 à 4, comprenant une étape consistant à ajouter un adsorbant carboné sphérique devant constituer une substance à étudier à une solution d'essai comprenant une substance à adsorber, et à permettre l'adsorption à une température prédéterminée pendant une durée prédéterminée sous agitation, et une étape consistant à mesurer la concentration ou la quantité de chaque substance à adsorber restant dans la solution d'essai sans l'adsorbant carboné sphérique.

6. Procédé de test selon la revendication 5, dans lequel la concentration de chaque substance à adsorber dans la solution d'essai avant addition de l'adsorbant carboné sphérique est de 0,05 à 0,5 mg/ml.

7. Procédé de test selon la revendication 6, dans lequel la concentration de chaque substance à adsorber dans la solution d'essai avant addition de l'adsorbant carboné sphérique est de 0,05 à 0,2 mg/ml.

8. Procédé de test selon la revendication 7, dans lequel la concentration de chaque substance à adsorber dans la solution d'essai avant addition de l'adsorbant carboné sphérique est de 0,1 mg/ml.

9. Procédé de test selon l'une quelconque des revendications 5 à 8, dans lequel la solution d'essai est obtenue en dissolvant chaque substance à adsorber dans un tampon au phosphate à un pH de 6 à 8.

10. Procédé de test selon la revendication 5, dans lequel l'adsorbant carboné sphérique est ajouté en une quantité de 10 à 300 mg pour 50 ml de la solution d'essai.

11. Procédé de test selon la revendication 10, dans lequel l'adsorbant carboné sphérique est ajouté en une quantité de 40 à 120 mg pour 50 ml de la solution d'essai.

12. Procédé de test selon la revendication 11, dans lequel l'adsorbant carboné sphérique est ajouté en une quantité de 50 mg ou 100 mg pour 50 ml de la solution d'essai.

13. Procédé de test selon l'une quelconque des revendications 10 à 12, dans lequel le volume de la solution d'essai est de 10 à 300 ml.

14. Procédé de test selon la revendication 13, dans lequel le volume de la solution d'essai est de 30 à 100 ml.

15. Procédé de test selon la revendication 14, dans lequel le volume de la solution d'essai est de 50 ml.

16. Procédé de test selon l'une quelconque des revendications 5 à 15, dans lequel le temps d'adsorption est de 1 à 6 h, et la température est de 10 à 50 °C.
